# EUROPEAN PATENT APPLICATION

(11) **EP 4 803 048 A1**
(43) Date of publication of application: **09.09.2026**
(21) Application number: 26157076.6
(22) Date of filing: 06.02.2026
(51) Int. Cl.: A61F 2/42, A61F 2/30

(54) **CONVERTIBLE JOINT IMPLANT FOR HEMI-ARTHROPLASTY**

(30) Priority: 03.03.2025 EP 25161388
(71) Applicant: LOCI Orthopaedics Limited, Galway, H91 KT67 (IE)
(72) Inventor: BOLAND, Brendan, Galway (IE); MANGAN, Fiona, Galway (IE); CLARKE, Gerry, Galway (IE); STOCKMANS, Filip, Galway (IE); LADD, Amy L., Galway (IE); WEISS, Arnold-Peter C., Galway (IE); COPE, Aiden, Galway (IE)
(74) Representative: Weldon O'Brien Ltd.

(57) **Abstract**

An implant apparatus (1, 100, 200) has a distal component (10, 101, 201) with a stem for intramedullary insertion in a distal bone such as the metacarpal. A proximal component is for sliding engagement on the trapezium as is known in the art. The apparatus also has a replacement proximal component (30, 110, 210) which can be releasably connected to the distal component by threads (23, 15, 104, 115, 204, 215) so that it may replace the original proximal component during revision surgery to provide a different manner of hemi arthroplasty articulation on a proximal bone. For example, after a trapeziectomy, the replacement may extend as far proximally as the scaphoid bone for translational movement on this bone, whereas the original proximal component engaged a surface of the trapezium with translational movement. The replacement proximal component may be long enough to span the longitudinal dimension of the trapezium that has been removed by use of a bridging part (20) which is long enough to span most of this distance.

## Description

The present invention relates to conversion of joint implants such as hip, shoulder, wrist, ankle, elbow, and Carpometacarpal (CMC) joint implants from one hemi-arthroplasty to a different hemi-arthroplasty. An example is at the trapezo-metacarpal joint.

EP3413843B (NUIG) discloses an implant with a stem for the metacarpal and a proximal saddle for sliding on the trapezium. The saddle has a neck extending distally and terminating in a ball in a socket in the proximal end of the stem. EP3914193B (Loci Orthopaedics Ltd) describes an approach in which the stem has a flexible insert and the ball is inserted in a socket in the insert in the stem, and the insert has a flange to provide optimal contact for extreme positions of the saddle. Both of these approaches provide a hemi arthroplasty implant, as the proximal part (the saddle) slides on the trapezium.

EP3914194B (Loci Orthopaedics Ltd.) describes an approach in which the hemi arthroplasty implant may be converted to a total arthroplasty. In revision surgery the proximal part with the saddle is removed, and a replacement part is screwed into a threaded socket in the stem, and a ball at the proximal end of the replacement part is engaged in a socket of a cup embedded in the trapezium. After the revision surgery it is a total arthroplasty because the stem is embedded in the metacarpal and the cup is embedded in the trapezium Also, the rotational part of the articulation is then about a socket in the trapezium, not about a socket in the stem in the metacarpal as it was originally.

The thumb base joint, also referred to as the first carpometacarpal (CMC1) joint or trapeziometacarpal joint, consists of the first metacarpal bone of the hand and the trapezium bone of the wrist. Osteoarthritis of the thumb base joint is most often graded using the Eaton-Littler classification. Grade I is the least severe with only minor joint structural changes, while Grade IV is the most severe to the extent that there are joint changes in the neighbouring scapho-trapezial joint. For such severe osteoarthritis, given the involvement of the scapho-trapezoidal joint, an intervention involving only the trapezo-metacarpal joint would not be clinically successful. Therefore, these patients usually undergo a trapeziectomy i.e., removal of the trapezium bone. The removal of the trapezium leaves a void, and many surgeons harvest the flexor carpi radialis (FCR) tendon and roll it up to an "anchovy" to fill the void. The aim of such products and procedures is to fill the void created to maintain the length, and therefore strength of the thumb, and preventing subsidence and impingement of the metacarpal on surrounding bones.

However, studies show that approx. 10% of patients are not satisfied with the outcomes after trapeziectomy (Saab M, Chick G. Trapeziectomy for trapeziometacarpal osteoarthritis. Bone Jt Open. 2021 Mar;2(3):141-149. doi: 10.1302/2633-1462.23.BJO-2020-0188.R1. PMID: 33650434; PMCID: PMC8009903).

The invention is directed towards providing more versatility in modification/conversion of a hemi arthroplasty implant after it has been implanted but is no longer clinically optimal.

### Summary of the Invention

The invention provides an implant apparatus as set out in claim 1, a kit as set out in claim 9.

We describe a bone joint implant apparatus comprising:
a distal component having an intramedullary stem for insertion in a distal bone, and
a proximal component having a proximal articular surface for translational engagement on a distal surface of a proximal bone, and
a replacement proximal component having a different configuration and being configured for translational engagement on a distal surface of a proximal bone, and
a coupler comprising a distal component coupler part and a proximal component coupler part for releasably coupling the proximal component to the distal component wherein the proximal and distal components are releasably secured to each other to allow replacement of the proximal component during revision surgery by the replacement proximal component.

In some preferred examples, the coupler comprises a discrete bridging part for releasably securing the distal component to the proximal component.

In some preferred examples, the proximal component comprises a socket engaging a ball at a proximal end of the discrete bridging part.

In some preferred examples, the socket is in a sleeve extending distally from a saddle having a proximal articular surface.

In some preferred examples, the discrete bridging part comprises a bolt having the ball at its proximal end and threads at its distal end.

In some preferred examples, the coupler comprises fasteners on the proximal and distal components for direct coupling together.

In some preferred examples, the fasteners are threaded fasteners.

In some preferred examples the distance between the proximal component proximal surface and a centre of rotation of the ball is in the range of 1 mm to 5mm, optionally 2 mm to 4 mm.

We also describe a kit comprising an implant apparatus of any example described herein, and a plurality of replacement proximal components of different configurations.

In some preferred examples, at least two of said proximal components are different in terms of their longitudinal proximal-distal dimension.

In some preferred examples, at least two of said proximal components are different in terms of shape of the proximal surface.

We also describe a method of performing revision surgery using an implant apparatus of any example described herein, in which the distal component intramedullary stem is inserted in a distal bone, the method comprising the steps of:
separating the proximal component from the distal component by releasing the coupler parts,
providing a replacement proximal component with a coupler part, said replacement proximal component having a configuration better suited to the patient, and
replacing, using the coupler parts, the proximal component with the replacement proximal component.

Such revision surgery may be performed after progression of arthritis in the patient, for example.

In some examples the distal bone is the metacarpal and the proximal bone is the trapezium or the scaphoid. In this case the method may performed after a trapeziectomy, in which the replacement proximal component is configured for translational movement on a distal surface of the scaphoid.

In some cases, the coupler comprises a discrete bridging part for releasably securing the distal component to the proximal component, and the proximal component comprises a socket engaging a ball at a proximal end of the discrete bridging part.

In one example method the socket is in a sleeve extending distally from a saddle having a proximal articular surface, and the discrete bridging part comprises a bolt having the ball at its proximal end and threads at its distal end. The bolt may be provided of any desired length to suit the surgical situation.

### Detailed Description of the Invention

The invention will be more clearly understood from the following description of some embodiments thereof, given by way of example only with reference to the accompanying drawings in which:
Fig. 1(a) is a cross-sectional view of a distal component of an implant having a stem for intramedullary insertion in the first metacarpal, and a side view of a replacement part which is inserted during revision surgery, Fig. 1(b) is a perspective view of a proximal component for insertion during the revision surgery, Fig. 1(c) is a front view of the proximal component, and Fig. 1(d) is a cross-sectional view in the direction of the arrows A-A of Fig. 1 (c),
Fig. 2 is a side view of an alternative implant apparatus of the invention, suitable for conversion to different hemi arthroplasty implant,
Fig. 3 is a side view of the implant of Fig. 2, with the proximal and distal components separated and internal details of the distal component shown, and Fig. 4 is a side view showing dimensional details of the proximal surface of the proximal component, and
Fig. 5 is a side view of an alternative implant, with the proximal and distal components separated and internal details of the distal component shown, and Fig. 6 is a side view showing dimensional details of the proximal surface of the proximal component.

### Thumb Joint, Conversion from Hemi Arthroplasty Implant to a Different Hemi Arthroplasty implant after a Trapeziectomy

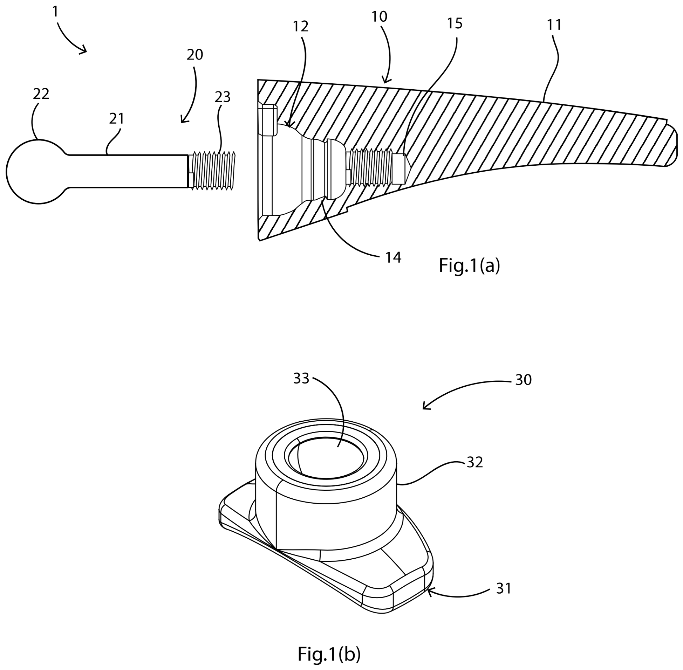

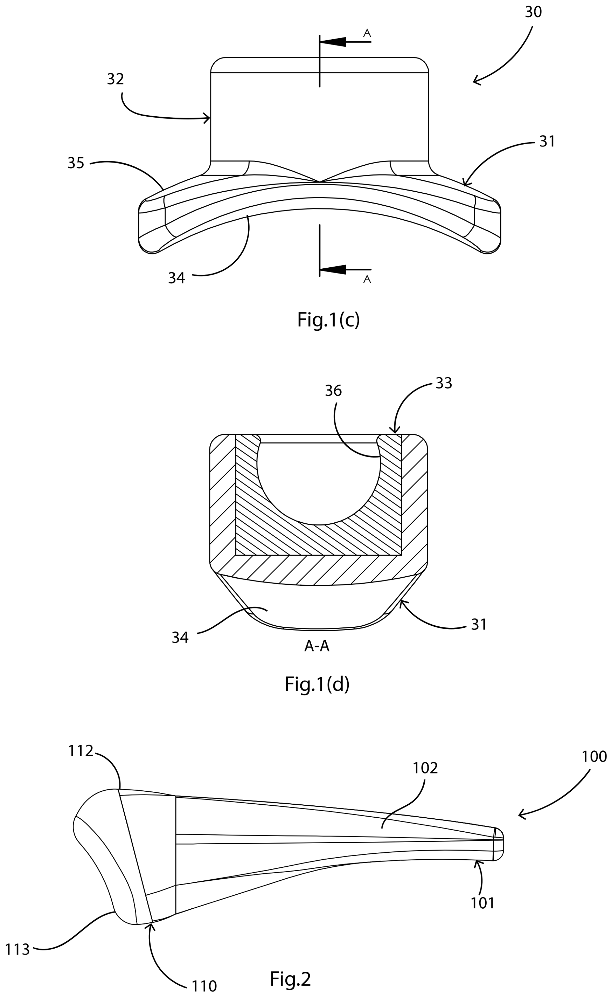

Referring to Figs. 1(a), 1(b), 1(c), and 1(d) an implant apparatus 1 comprises a distal component comprising a stem 10 for intramedullary insertion in the first metacarpal. The stem 10 has a metal body 11 extending distally for insertion in the intramedullary canal of the metacarpal. Within its proximal end there is a cavity 12 configured to receive an insert with a ball socket for receiving a ball of a hemi arthroplasty proximal implant component known in the art, especially as described in EP3914193B. The cavity 12 also includes annular grooves 14 for anchoring the insert, also as known in the art. In addition, the cavity 12 includes a threaded socket 15 for receiving, upon conversion with removal of the insert, a replacement proximal component including a bridging component 20. The bridging component 20 has a stem 21 with a distal threaded end 23 for engaging the stem's threaded socket 15, and a proximal end with a ball 22. The latter is in this case for engagement with an implant proximal component bone-contacting part 30, shown in Figs. 1(b), 1(c), and 1(d), having a saddle 31 shaped for sliding on the scaphoid. The proximal component part 30 also has a cylindrical housing 32 containing an insert 33 with a ball socket 36 configured to receive the ball 22 in a snap fit. The saddle 31 has a proximal bone-engaging surface 34 and a distal surface 35.

Hence in use an implant may provide a hemi arthroplasty on the trapezium for a period of time, and subsequently a modified implant may provide a hemi arthroplasty on the scaphoid after removal of the trapezium. In the latter case the bridging component 20 spans the void left by removal of the trapezium and the proximal component bone-contacting part has a different proximal articular surface.

Post trapeziectomy, the stem remains in the metacarpal and the replacement proximal component 30 is shaped at its saddle 31 on the proximal side to move translationally and articulate over the distal surface of the scaphoid. The proximal component part 30 may be metal, polymer, PEEK, PyC or any metal with the appropriate mechanical features. The socket may be metal or polymer, chosen for suitable interfacing with the materials used for the other elements. For example, a metallic ball of the neck may be best articulating with a medical grade polymer socket.

The invention avoids needing to place a fixed socket in the scaphoid bone. Also, the points of motion, i.e. the ball 22 rotation and the translational movement of the saddle 31, are close together.

In one example the distance between the scaphoid distal surface and the ball 22 centre of rotation is approximately 2 mm to 4 mm. This provides a low risk of "jack-knifing" of the implant.

In general, it is preferred that the closest distance between a proximal surface of the saddle 31 and a centre of the ball 22 (which is also the centre of the socket 33) is in the range of 1 mm to 5 mm, and preferably 2 mm to 4 mm.

Also, the movement of the point of rotation proximally to the distal surface of the scaphoid may have biomechanical benefits for stability in a less mobile part of the wrist.

The saddle 31 can be of a desired thicknesses depending on the anatomy of the joint and the physiological laxity of surrounding tissue. The ball may be larger or smaller depending on the size of the joint. A larger ball will increase the surface area over which wear occurs but will also increase the "jump distance" i.e. the distance the ball must move before dislocating from the socket. Larger balls are less likely to dislocate. In some examples a range of different sizes of proximal component 30 and indeed bridging component 20 are supplied as a conversion kit, allowing the surgeon to choose the optimum size.

The socket 33 thickness may be chosen to optimise wear patterns. The implant apparatus would maintain the length and hence strength of the thumb, and would maintain the position of the metacarpal, preventing metacarpal impingement on surrounding structures.

The modularity of the apparatus allows for optimised patient construct tailoring as modularity can be based on neck length, ball size, socket size, head size, head thickness, and/or material choice. While in this embodiment the socket 33 is central on the proximal component 30, it may alternatively be offset, based on patient biomechanics. Additionally, while the neck is shown as being straight, it may alternatively be offset, based on patient biomechanics.

In other clinical scenarios, patients may undergo a partial trapeziectomy. In such cases many patients have a grade of osteoarthritis less than Grade IV (four). The benefits as outlined above are all still applicable in cases of post-partial trapeziectomy. As noted in some studies, patients who undergo partial trapeziectomy may have higher complications than those that undergo full trapezium, hence this cohort may benefit the most from the invention. (Ganhewa AD, Wu R, Chae MP, Tobin V, Miller GS, Smith JA, Rozen WM, Hunter-Smith DJ. Failure Rates of Base of Thumb Arthritis Surgery: A Systematic Review. J Hand Surg Am. 2019 Sep;44(9):728-741.e10. doi: 10.1016/j.jhsa.2019.05.003. Epub 2019 Jun 28. PMID: 31262534.) More refs can be supplied.

### Thumb Joint Hemi Arthroplasty without Trapeziectomy

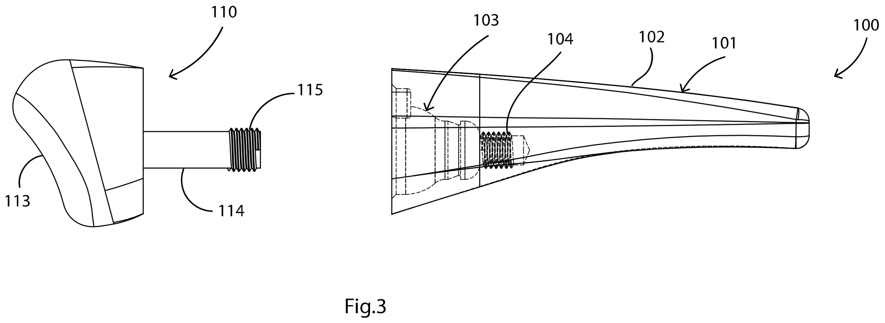

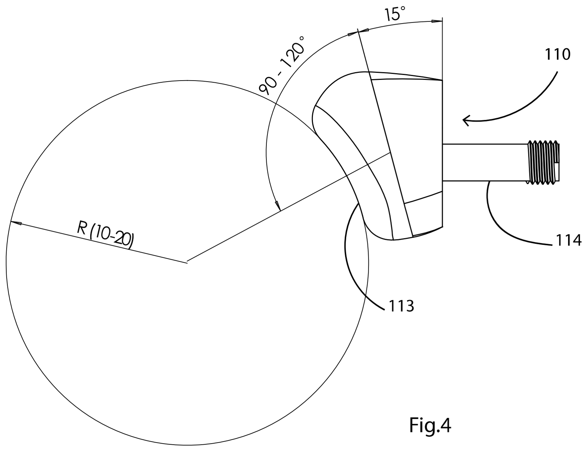

Referring to Figs. 2 to 4 an implant apparatus comprises a single device 100 with two components which are removably attached to each other. There is a distal component 101 which is a metacarpal stem, and a proximal component 110 which is for sliding/translational engagement on the trapezium. The proximal component 110 comprises a body 112 with a proximal saddle shaped surface 113 for sliding/translational engagement on the distal surface of the trapezium.

The components 101 and 110 are both threaded so that a male threaded fastener part on one is engaged by rotation to a female threaded fastener part on the other component. In this way, the surgeon may perform a revision by un-coupling the proximal component 110 and attaching a replacement proximal component which may be different in terms of:
The longitudinal dimension, even so far as possibly spanning the void left after a trapeziectomy or simply adjusting to a different longitudinal anatomical dimension in the patient.
Contours of the proximal surface 111 to accommodate trapezium wear or subluxation.

In this example the coupling is by way of the distal component having a cavity 103 at the distal most end of which there is a threaded socket 104. The proximal component 110 comprises a bolt 114 with a threaded distal end 115 for engagement in the socket 104.

The apparatus 100 proximal surface 113 has a radius of curvature in the range of 10 mm to 20 mm and an offset from a plane normal to the bolt 114 (the longitudinal axis) in the range of 105° to 135°. However, these are only examples to illustrate the advantage that the proximal component may have any desired configuration to suit clinical needs and can be inserted during revision surgery without needing to remove the stem in the first metacarpal.

The apparatus 100 is suitable for patients affected with osteoarthritis, with the proximal component providing a modular hemi-arthroplasty that articulates with the trapezium. The distal (stem) component may be of a size appropriate to the medullary canal of the first metacarpal where this is placed and can have an element such as threads to receive a head (proximal) component and can be made of a material most suitable to a bone interface, for example metal.

The replacement proximal component, being modular, allows the angle of articulation with the trapezium to be changed. In patients with aligned joints this can be different to patients with subluxed or misaligned joints. The distance from the stem component 101 to the articular surface 113 can be changed during revision surgery by replacement of the proximal component, providing proximal to distal sizing options based on patient anatomy, joint biomechanics, and joint laxity. This may also be modifiable in the case of a partial or total trapeziectomy, where the proximal component articulates on a resected trapezium, or on the scaphoid in the absence of a trapezium.

The distal component may provide a stems Size in the range of A to E (of increasing length and diameter). The proximal component may have a head thickness of Short, Medium, Long (of increasing depth). The articular angle may be Steep, Medium, Shallow (of decreasing angulation). The material of either of the components may be Titanium, Cobalt Chrome, PEEK, UHMPPE, and/or Pyrocarbon.

The materials of the component may be different based on optimal article properties with bone, for example polymer, metal, and/or pyrocarbon.

The components may be joined by threads or other mechanisms. An example is a locking mechanism configured such that the proximal surface 113 "stops" in the correct place with alignment to the stem 101. In this case the threads match so that when the head 110 is threaded in, the head comes to a final stop position with the head aligned to the stem 101. Double threads or star threads may be optimised to ensure the appropriate stop position of the head.

In this invention we describe replacement of one hemi arthroplasty proximal component with another for optimal clinical conditions.

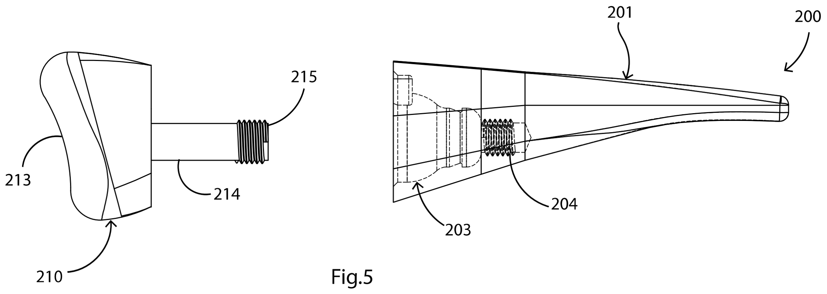

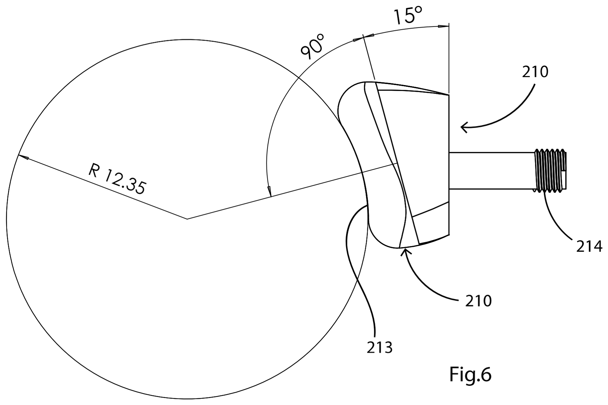

Referring to Figs. 5 and 6 an alternative implant 200 comprises a distal component 201 with a proximal cavity 203 having a threaded socket 204 performing the same functions as the parts 103 and 104 of the implant of Figs. 2 to 4. There is a proximal component 210 with a bolt 214 and a threaded end 215, and this component has a proximal articular surface 213. In this case there is a radius of curvature of the articular surface of 12.5 mm.

The embodiments 100 and 200 provide for versatility in choice of proximal articular surfaces for sliding on the trapezium. However, it is envisaged that the body of the proximal component may have a longitudinal dimension to fill the void caused by removal of the trapezium, and the proximal articular surface is configured to slide on the scaphoid.

These examples illustrate that because the proximal component can be decoupled from the distal component there are a wide range of options for conversion following an initial surgery. This provides for relatively simple revision surgery in the case of osteoarthritis of the joint. If there is severe osteoarthritis an intervention involving only the trapezo-metacarpal joint would not be clinically successful and the invention provides for removal of the trapezium with bridging of the void. This avoids need for surgeons to harvest the flexor carpi radialis (FCR) tendon and rolling it up to fill the void.

Components of embodiments can be employed in other embodiments in a manner as would be understood by a person of ordinary skill in the art. The invention is not limited to the embodiments described but may be varied in construction and detail.

## Claims

1. A bone joint implant apparatus (1, 100) comprising:
a distal component (10, 101, 201) having an intramedullary stem for insertion in a distal bone, and
a proximal component having a proximal articular surface for translational engagement on a distal surface of a proximal bone,
a replacement proximal component (20, 30, 110) having a different configuration and being configured (34, 113, 213) for translational engagement on a distal surface of a proximal bone, and
a coupler (20, 114, 214) comprising a distal component coupler part (15, 104, 204) and a proximal component coupler part (20, 114,214) for releasably coupling the proximal component to the distal component wherein the proximal and distal components are releasably secured to each other to allow replacement of the proximal component during revision surgery by the replacement proximal component.

2. An implant apparatus as claimed in claim 1, wherein the coupler comprises a discrete bridging part (20) for releasably securing the distal component to the proximal component.

3. An implant apparatus as claimed in claim 2, wherein the replacement proximal component (30) comprises a socket (33) engaging a ball (22) at a proximal end of the discrete bridging part (30).

4. An implant apparatus as claimed in claim 3, wherein the socket (33) is in a sleeve (32) extending distally from a saddle having a proximal articular surface (34).

5. An implant apparatus as claimed in any of claims 2 to 4, wherein the discrete bridging part comprises a bolt (21) having the ball (22) at its proximal end and threads (23) at its distal end.

6. An implant apparatus as claimed in claim 5, wherein the distance between the proximal component (30) proximal surface (34) and a centre of rotation of the ball (22) is in the range of 1 mm to 5mm, optionally 2 mm to 4 mm.

7. An implant apparatus as claimed in claim 1, wherein the coupler comprises fasteners (104, 115, 204, 215) on the proximal and distal components (101, 201, 110, 210) for direct coupling together.

8. An implant apparatus as claimed in claim 7, wherein the fasteners are threaded fasteners (104, 115).

9. A kit comprising an implant apparatus as claimed in any preceding claim, and a plurality of replacement proximal components of different configurations.

10. A kit as claimed in claim 9, wherein at least two of said replacement proximal components are different in terms of their longitudinal proximal-distal dimension.

11. A kit as claimed in claim 9 or claim 10, wherein at least two of said proximal components are different in terms of shape of the proximal articular surface (34, 113, 213).
